# EUROPEAN PATENT APPLICATION

(11) **EP 4 541 405 A1**
(43) Date of publication of application: **23.04.2025**
(21) Application number: 22947466.3
(22) Date of filing: 29.06.2022
(51) Int. Cl.: A61M 25/01, A61B 18/14, A61N 1/05

(54) **INTERVENTIONAL APPARATUS FOR IMPLANTING ELECTRODE INTO BRAIN BY BLOOD VESSEL, AND MEDICAL DEVICE**

(30) Priority: 20.06.2022 CN 202210697115
(71) Applicant: Shanghai Stairmed Technology Co., Ltd., Shanghai 200131 (CN)
(72) Inventor: ZHAO, Zhengtuo, Shanghai 200031 (CN); LI, Xue, Shanghai 200031 (CN); WANG, Xingzhao, Shanghai 200031 (CN)
(74) Representative: Cooley (UK) LLP
(86) International application number: PCT/CN2022/102207
(87) International publication number: WO 2023/245703

(57) **Abstract**

The present disclosure relates to an interventional device for implanting an electrode into the brain via a blood vessel, the blood vessel having a blood vessel channel and a blood vessel wall, characterized in that the interventional device comprises: a first guide member, the distal end of which can move distally in the blood vessel channel and establish a first support point in the blood vessel channel; a second guide member, the distal end of which can pass through the first guide member to move distally in the blood vessel channel and establish a second support point in the blood vessel channel, so that the second guide member extends in an arc shape between the first support point and the second support point; and a puncture kit, the puncture kit being used to carry the electrode, and the distal end of the puncture kit being able to pass through the first guide member to move distally in the blood vessel channel and puncture the blood vessel wall in a target puncture area between the first support point and the second support point, thereby implanting the carried electrode into the brain. Furthermore, the present disclosure relates to a medical apparatus.

## Description

### FIELD OF THE INVENTION

The present disclosure relates to the field of medical devices, and more particularly, to an interventional device for implanting an electrode into brain via a blood vessel and a medical apparatus.

### BACKGROUND OF THE INVENTION

In the technical field of brain-computer interfaces, electrodes (such as flexible electrode wires) are widely used. Electrodes can be implanted in the brain to collect signals from neurons near the electrodes. The current solution for implanting electrodes in the brain is to first perform bone removal and window opening in the skull through surgery, open the dura mater to expose the target implantation area on the cerebral cortex, and then implant the electrodes by machine or manually. However, such a solution of implanting electrodes in the brain will inevitably lead to nerve cell damage in non-target areas and may even cause brain damage when the target area of implantation is a functional area deep in the brain instead of the cortex. Therefore, it is necessary to find a novel solution for implanting electrodes into the brain, in particular deep in the brain.

### SUMMARY OF THE INVENTION

Provided below is a brief summary of the present disclosure in order to provide a basic understanding of some aspects of the present disclosure. However, it should be understood that this summary is not an exhaustive overview of the present disclosure. It is not intended to identify key or important parts of the present disclosure, nor is it intended to limit the scope of the present disclosure. Its purpose is merely to present some concepts about the present disclosure in a simplified form as a prelude to the more detailed description that is presented later.

According to a first aspect of the present disclosure, there is provided an interventional device for implanting an electrode into the brain via a blood vessel, the blood vessel having a vascular channel and a blood vessel wall, wherein the interventional device comprises: a first guide member, the distal end of which can move distally in the vascular channel and establish a first support point in the vascular channel; a second guide member, the distal end of which can pass through the first guide member to move distally in the vascular channel and establish a second support point in the vascular channel, so that the second guide member extends in an arc shape between the first support point and the second support point; and a puncture kit, the puncture kit being used to carry the electrode, and the distal end of the puncture kit being able to pass through the first guide member to move distally in the vascular channel and puncture the blood vessel wall in a target puncture area between the first support point and the second support point, thereby implanting the carried electrode into the brain.

According to a second aspect of the present disclosure, there is provided an interventional device for implanting an electrode into the brain via a blood vessel, the blood vessel comprising a first blood vessel segment and a second blood vessel segment extending in different directions, characterized in that the interventional device comprises: a first guide member, the distal end of which is capable of moving distally in a blood vessel channel of the first blood vessel segment and reaching a first position, the first position being located in the first blood vessel segment and near a connection between the first blood vessel segment and the second blood vessel segment; a second guide member, the distal end of which is capable of moving distally in the first guide member and continuing to move distally after reaching the distal end of the first guide member, so as to pass through the connection between the first blood vessel segment and the second blood vessel segment and enter the second blood vessel segment, thereby reaching and being fixed at a second position; and a puncture kit, the puncture kit being used to carry the electrode, and the distal end of the puncture kit being capable of moving distally in the first guide member and continuing to move distally after reaching the distal end of the first guide member, so as to puncture the blood vessel wall between the first position and the second position, thereby implanting the carried electrode into the brain.

According to a third aspect of the present disclosure, there is provided an interventional device for implanting an electrode into the brain via a blood vessel, wherein the blood vessel comprises a first blood vessel segment and a second blood vessel segment that are connected and extend in different directions, the connection between the first blood vessel segment and the second blood vessel segment being near the brain, and the interventional device comprises: a first catheter, the first catheter being configured in such a way that its distal end can reach and float at a first position located in the first blood vessel segment, and the opening of the distal end of the first catheter is substantially oriented toward a first direction, the first position being located near the connection, and the first direction being a direction in which the first blood vessel segment extends near the connection; a stent assembly, the stent assembly comprising a stent located at the distal end and a stent push rod connected to the stent, the stent assembly being configured in such a way that the stent can reach the distal end of the first catheter along the lumen of the first catheter, pass through the connection and enter the second blood vessel segment, and be fixed at a second position located in the second blood vessel segment so that the stent push rod can extend along the branch direction of the blood vessel between the first position and the second position, and the opening of the distal end of the first catheter can be deflected to a second direction under the action of the stent push rod, the second direction being the direction of extension of the second blood vessel segment near the connection; a third catheter, the third catheter being configured in such a way that its distal end can reach and extend from the distal end of the first catheter along the lumen of the first catheter to approach the puncture position on the blood vessel wall; and a puncture kit, the puncture kit is used to carry the electrode, and the puncture kit is configured in such a way that its distal end can approach the distal end of the third catheter along the lumen of the third catheter, wherein the third catheter is also configured in such a way that after the distal end of the puncture kit approaches the distal end of the third catheter, the distal end of the third catheter can puncture the blood vessel wall at the puncture position and reach outside the blood vessel without entering the brain, and the puncture kit is also configured in such a way that after the distal end of the third catheter reaches outside the blood vessel, it continues to move along the lumen of the third catheter and passes through the distal end of the third catheter to enter the brain, thereby implanting the carried electrode into the brain.

According to a fourth aspect of the present disclosure, there is provided an interventional device for implanting an electrode into the brain via a blood vessel, wherein the blood vessel comprises a first blood vessel segment and a second blood vessel segment that are connected and extend in different directions, the connection between the first blood vessel segment and the second blood vessel segment being near the brain, and the interventional device comprises: a first catheter, the first catheter being configured in such a way that its distal end can reach and be located at a first position in the first blood vessel segment, the first position being located near the connection; a stent assembly, the stent assembly comprising a stent located at the distal end and a stent push rod connected to the stent, the stent assembly being configured in such a way that the stent can reach the distal end of the first catheter along the lumen of the first catheter, pass through the connection to enter the second blood vessel segment, and be fixed at a second position in the second blood vessel segment, so that the stent push rod extends between the first position and the second position; a second catheter, the second catheter being configured in such a way that the second catheter is able to sleeve the stent push rod, and the distal end of the second catheter can reach and be located at a specific position between the first position and the second position; and a puncture kit, the puncture kit being used to carry the electrode, the puncture kit being configured in such a way that its distal end can reach the distal end of the second catheter along the lumen of the second catheter and continue to move forward after passing the distal end of the second catheter, so as to puncture the blood vessel wall at a puncture position corresponding to the specific position and enter the brain, thereby implanting the carried electrode into the brain.

According to a fifth aspect of the present disclosure, there is provided an interventional device for implanting an electrode into the brain via a blood vessel, wherein the blood vessel comprises a first blood vessel segment and a second blood vessel segment that are connected and extend in different directions, the connection between the first blood vessel segment and the second blood vessel segment being near the brain, and the interventional device comprises: a first catheter, the first catheter being configured in such a way that its distal end can reach and float at a first position located in the first blood vessel segment, and the opening of the distal end of the first catheter is substantially oriented toward a first direction, the first position being located near the connection, and the first direction being a direction in which the first blood vessel segment extends near the connection; a stent assembly, the stent assembly comprising a stent located at the distal end and a stent push rod connected to the stent, the stent assembly being configured in such a way that the stent can reach the distal end of the first catheter along the lumen of the first catheter, pass through the connection and enter the second blood vessel segment, and be fixed at a second position located in the second blood vessel segment so that the stent push rod can extend along the branch direction of the blood vessel between the first position and the second position, and the opening of the distal end of the first catheter can be deflected to a second direction under the action of the stent push rod, the second direction being the direction of extension of the second blood vessel segment near the connection; and an electrode guide needle, the distal end of the electrode guide needle being constructed to carry the electrode, the electrode guide needle being configured in such a way that its distal end can reach and extend from the distal end of the first catheter along the lumen of the first catheter, pass through the blood vessel wall at the puncture position on the blood vessel wall, and enter the brain, thereby implanting the carried electrode into the brain.

According to a sixth aspect of the present disclosure, a medical apparatus is provided, comprising the interventional device for implanting an electrode into the brain through a blood vessel according to the present disclosure.

Other features and advantages of the present disclosure will become more apparent from the following detailed description of exemplary embodiments of the present disclosure with reference to the accompanying drawings.

### BRIEF DESCRIPTION OF THE DRAWINGS

The accompanying drawings, which constitute a part of the specification, illustrate embodiments of the present disclosure and, together with the description, are used to explain the principles of the present disclosure. The embodiments set forth in the accompanying drawings are illustrative and exemplary in nature and are not intended to limit the present disclosure. The following detailed description of exemplary embodiments may be clearly understood when read in conjunction with the accompanying drawings below, wherein like structures are indicated by like ls, and wherein:
FIG. 1 is a perspective view that schematically shows a human brain;
FIG. 2 is a perspective view that schematically shows an interventional device for implanting an electrode into the brain through a blood vessel according to one or more exemplary embodiments of the present disclosure;
FIG. 3 is a perspective view that schematically shows an interventional device for implanting an electrode into the brain through a blood vessel according to one or more exemplary embodiments of the present disclosure, wherein the electrode has been implanted into the brain;
FIGS. 4A-4C respectively show three exemplary electrode guide needles for an interventional device used for implanting an electrode into the brain through a blood vessel according to one or more exemplary embodiments of the present disclosure;
FIGS. 5A-5C respectively show three exemplary occluders equipped for an interventional device for implanting an electrode into the brain through a blood vessel according to one or more exemplary embodiments of the present disclosure;
FIGS. 6A-6G are schematic diagrams that schematically show the use of an interventional device for implanting an electrode into the brain through a blood vessel according to one or more exemplary embodiments of the present disclosure;
FIGS. 7A-7F are perspective diagrams that schematically show the use of an interventional device for implanting an electrode into the brain through a blood vessel according to one or more exemplary embodiments of the present disclosure;
FIGS. 8A and 8B are schematic diagrams schematically illustrating a puncture kit according to an embodiment of the present disclosure.

### DETAILED DESCRIPTION OF THE INVENTION

Various exemplary embodiments of the present disclosure will be described in detail below with reference to the accompanying drawings. It should be noted that the relative arrangement of components and steps, the numerical expressions, and numerical values set forth in these embodiments do not limit the scope of the present disclosure unless specifically stated otherwise.

The following description of at least one exemplary embodiment is merely illustrative in nature and is in no way intended to limit the present disclosure and its application or uses. That is, the structures and methods herein are shown in an exemplary manner to illustrate different embodiments of the structures and methods in the present disclosure. However, those skilled in the art will appreciate that they are merely illustrative of exemplary, instead of exhaustive, ways in which the present disclosure may be implemented. Furthermore, the accompanying drawings are not necessarily to scale, and some features may be enlarged to show details of specific components.

In addition, technologies, methods, and apparatuses known to those skilled in the relevant field may not be discussed in detail, but where appropriate, such technologies, methods, and apparatuses should be considered part of the authorized specification.

In all examples shown and discussed herein, any specific values should be interpreted as merely exemplary and not as limiting. Therefore, other examples of the exemplary embodiments may have different values.

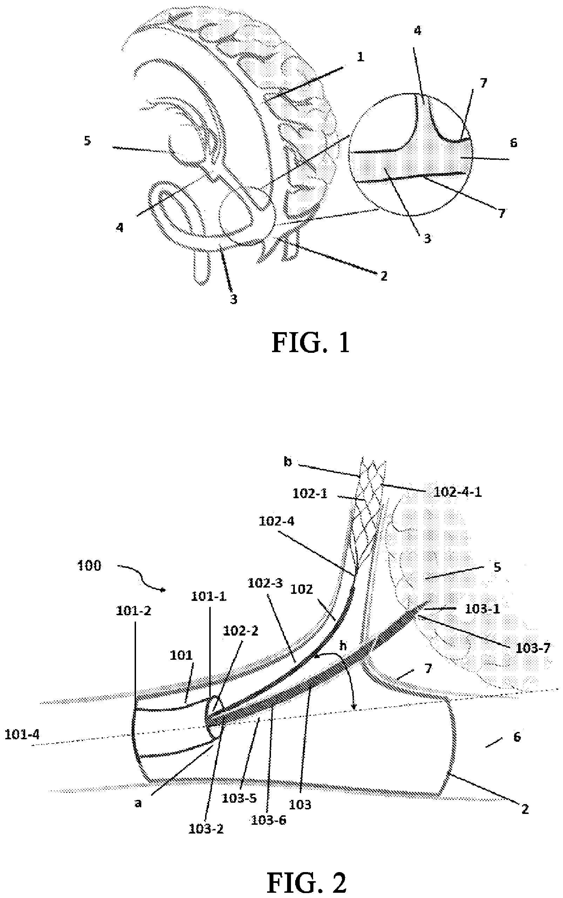

FIG. 1 schematically shows a human brain.

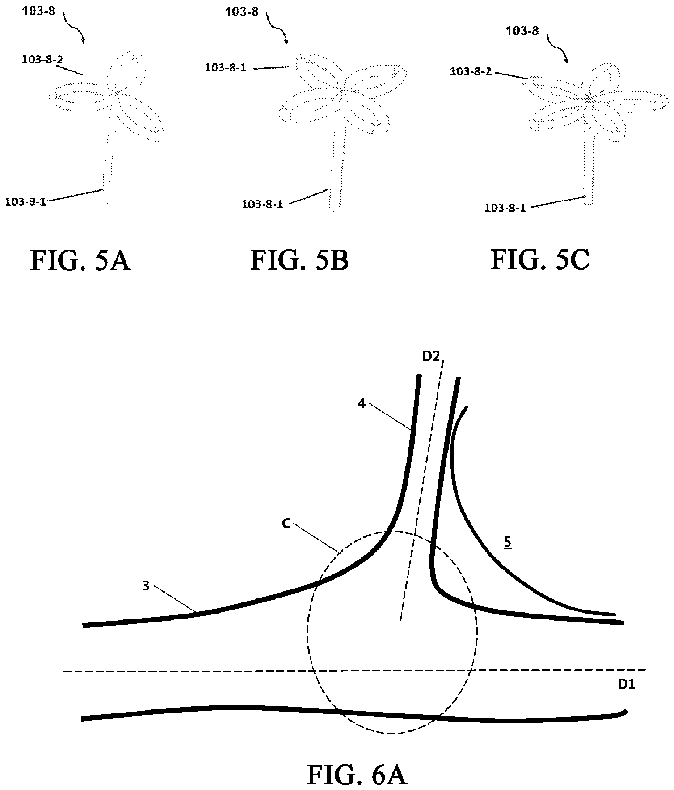

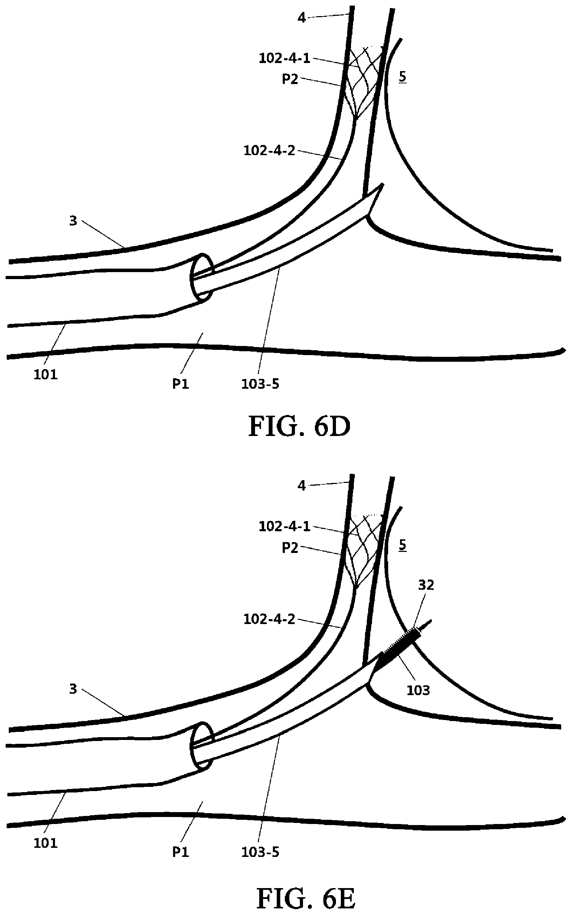

As shown in FIG. 1, in the present disclosure, in order to facilitate the explanation of the technical solution, the human brain is schematically divided into a superficial brain region 1 located in the shallow layer (surface layer) of the brain and a deep brain region 5 located in the deep layer of the brain. Superficial brain region 1 may include the cerebral cortex (cerebral cortex). In the cerebral cortex, there are functional areas such as movement and vision. Deep brain region 5 may include deep brain nuclei, which include important functional areas such as the nucleus accumbens, caudate nucleus, putamen, globus pallidus, red nucleus, dentate nucleus, ventroposterior lateral nucleus, ventroposterior medial nucleus, and ventroposterior nucleus. Furthermore, it can be understood that there are a plurality of blood vessels in the superficial brain region 1 and the deep brain region 5 of the human brain. The plurality of blood vessels may include a main blood vessel 3, branch blood vessels 4, and a transition region 2 between the main blood vessel 3 and the branch blood vessels 4. The branch blood vessels 4 extend from the main blood vessel 3 as branches, and the connection between the main blood vessel and the branch blood vessels (refer to the connection C in FIG. 6A) are located near the brain (e.g., the deep brain region 5). Each blood vessel has a blood vessel channel 6 and a blood vessel wall 7.

Referring to FIG. 1, those skilled in the field of brain-computer interface can understand that the technical solution of implanting the electrode 32 through craniotomy described at the beginning of the specification is only applicable to implanting the electrode 32 into the superficial brain area 1, and is not applicable to implanting the electrode 32 into the deep brain area 5. In addition, such a technical solution requires craniotomy and deep brain implant surgery, which not only causes serious trauma to patients but also brings a huge workload to doctors.

For this reason, the present disclosure provides an interventional device 100 for implanting an electrode 32 into the brain through a blood vessel (e.g., a cerebral vein, etc.). The interventional device can implant the electrode 32 into the brain of a living body(e.g., a human body or an animal body) through at most two vascular punctures, such as a jugular vein puncture and an intracranial vein puncture. This not only significantly reduces trauma to a patient (only two vascular puncture openings), but also reduces the workload of doctors. Furthermore, since blood vessels are distributed in almost all areas of the brain, the electrode 32 can be implanted in almost any area of the brain through corresponding blood vessels. Taking the subthalamic nucleus (STN) in the deep brain region 5 as an example, the interventional device 100 can establish an electrode implantation path starting from the jugular vein, through the sigmoid sinus to the transverse sinus, then through the confluence of sinuses to the straight sinus, and finally through the Galen vein and then the thalamostriate vein, to implant the electrode 32 into the subthalamic nucleus. Furthermore, the electrodes implanted using the interventional device 100 of the present disclosure may have both the function of collecting neuron signals and the function of neuron stimulation. Therefore, the interventional device 100 according to the present disclosure not only supports the acquisition of neuron signals and the stimulation of neurons in the superficial brain region 1, but also supports the acquisition of neuron signals and the stimulation of neurons in the deep brain region 5. In addition, according to the interventional device 100 disclosed in the present disclosure, the insertion direction of the puncture needle or the target puncture area in the puncture kit can be set or adjusted in a targeted manner by determining or adjusting the positions of the first support point a and the second support point b, thereby providing a relatively stable puncture track for the electrode guide needle 103 of the interventional device 100 and thus improving the accuracy of the implantation of the electrode 32.

An interventional device 100 for implanting an electrode 32 into the brain through a blood vessel according to various embodiments of the present disclosure will be described in detail below with reference to FIGS. 2 to 5C . It is understandable that an actual interventional device 100 may have other components, but in order to avoid obscuring the main points of the present disclosure, the other components are neither shown in the accompanying drawings nor discussed herein. It should also be noted that the mentioning of "distal end" or "distal side" herein refers to the end or side away from an operator/surgery performer (usually a doctor), and the mentioning of "proximal end" or "proximal side" refers to an end or side close to an operator/surgery performer (usually a doctor).

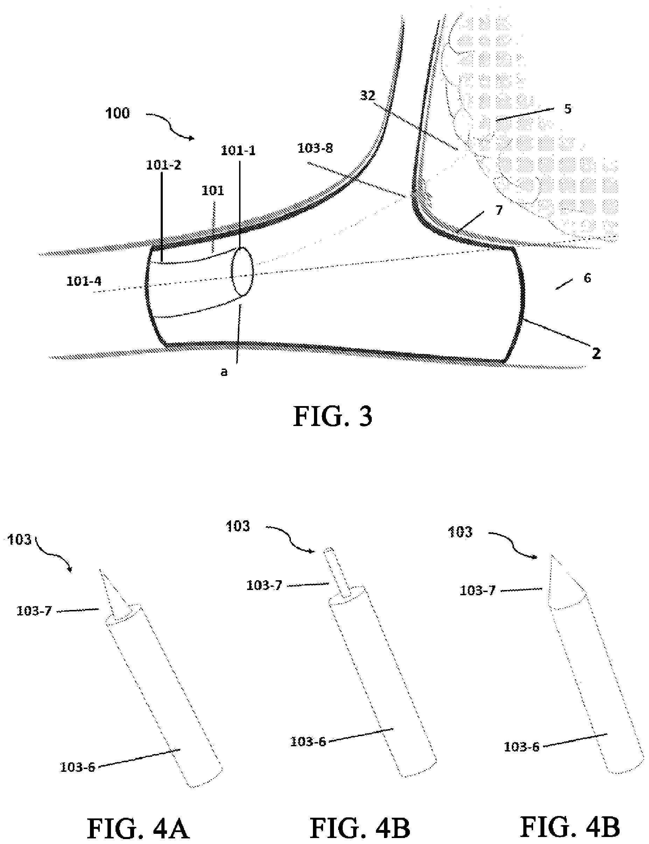

FIG. 2 and FIG. 3 schematically illustrate an interventional device 100 for implanting an electrode 32 into the brain through a blood vessel according to one or more exemplary embodiments of the present disclosure, wherein FIG. 2 is mainly used to illustrate the arrangement structure of the interventional device 100, and FIG. 3 is mainly used to illustrate the electrode 32 implanted into the brain and an occluder 103-8 for occluding the punctured blood vessel wall 7.

As shown in FIG. 2, an interventional device 100 for implanting an electrode 32 into the brain through a blood vessel according to the present disclosure may include a first guide member 101, a second guide member 102, and an electrode guide needle 103. The first guide member 101 may be configured to be able to be percutaneously inserted into a blood vessel of a living body (e.g., a human body, an animal body), and has a distal end 101-1 and a proximal end 101-2 opposite to the distal end 101-1. The distal end 101-1 of the first guide member 101 can move distally in a blood vessel channel 6 of the blood vessel and establish a first support point a in the blood vessel channel 6. Similar to the first guide member 101, the second guide member 102 also has a distal end 102-1 and a proximal end 102-2 opposite to the distal end 102-1, and the electrode guide needle 103 also has a distal end 103-1 and a proximal end 103-2 opposite to the distal end 103-1. The distal end 102-1 of the second guide member 102 can move distally through the first guide member 101 in the blood vessel channel 6 of the blood vessel and establish a second support point b in the blood vessel channel 6, so that the second guide member 102 extends in an arc shape between the first support point a and the second support point b. The distal end of the electrode guide needle 103 is constructed to carry the electrode 32, and the distal end of the electrode guide needle 103 is capable of passing through the first guide member 101, moving distally in the blood vessel channel 6, and puncturing the blood vessel wall 7 of the blood vessel in the target puncture area between the first support point a and the second support point b, thereby implanting the carried electrode 32 into the brain. In some embodiments, the electrode 32 may be a flexible electrode, such as an electrode wire. The electrode 32 should have sufficient length so that the proximal end of the electrode can be connected to an external data interface, thereby being able to transmit neuron signals collected from the brain to the data interface. It will be understood that although FIG. 2 illustrates the cross-sectional shape of the main body of the first guide member 101, the second guide member 102, and the electrode guide needle 103 as a circle, this is merely illustrative and not restrictive, and the main body of the first guide member 101, the second guide member 102, and the electrode guide needle 103 may have any suitable cross-sectional shape.

In some embodiments, as shown in FIG. 1, the blood vessels may include a main blood vessel 3 and a branch blood vessel 4. The first support point a may be established in the main blood vessel 3, and the second support point b may be established in the branch blood vessel 4. Here, the main blood vessel 3 may be a blood vessel having a diameter of approximately 3 mm to 6 mm, and the branch blood vessel 4 may be a blood vessel having a diameter of less than 2 mm to 3 mm. Thus, the interventional device 100 can establish a first support point a and a second support point b at a natural bifurcation structure of a blood vessel, so that the second guide member 102 extends in an arc shape between the first support point a and the second support point b. Considering that the second guide member 102 and the first guide member 101 form a nested structure, the arc-shaped extension of the second guide member 102 may cause the distal end 101-1 of the first guide member 101 to bend slightly toward the second guide member 102, for example, see FIGS. 2 and 3. Considering that the electrode guide needle 103 and the first guide member 101 also form a nested structure, when the first guide member 101 determines the extension direction of its main body, especially its distal end 101-1, the insertion direction of the electrode guide needle 103 or the target puncture area is also basically determined accordingly. Here, the needle insertion direction of the electrode guide needle 103 or the target puncture area is basically located in the angle area h formed by the central axis 101-4 of the first guide member 101 and the second guide member 102. In a case where the first support point a is located in the main blood vessel 3 and the second support point b is located in the branch blood vessel 4, the target puncture area may be located in the transition area 2 between the main blood vessel 3 and the branch blood vessel 4. Therefore, according to the interventional device 100 of the present disclosure, the insertion direction or the target puncture area of the electrode guide needle can be specifically set or adjusted by determining or adjusting the positions of the first support point a and the second support point b. Based on this, the interventional device 100 according to the present disclosure can provide a relatively stable puncture track for the electrode guide needle 103, thereby improving the accuracy of the electrode 32 implantation.

In some embodiments, the first guide member 101 can be configured as a support catheter, the outer diameter of the distal end 101-1 of the support catheter being substantially equal to the inner diameter of the blood vessel at the first support point a, so as to establish the first support point a in the blood vessel channel 6. In other embodiments, as shown in FIG. 2, the outer diameter of the distal end 101-1 of the support catheter may also be configured to be smaller than the inner diameter of the blood vessel at the first support point a. In addition, the support catheter may have a hollow structure to provide a first working channel (not shown) for the second guide member 102 and a second working channel (not shown) for the electrode guide needle 103 inside the support catheter. That is, the second guide member 102 and the electrode guide needle 103 are removably disposed in their respective working channels. As a result, the second guide member 102 and the electrode guide needle 103 can be guided independently of each other, within the support catheter. In some alternative embodiments, the support catheter may also provide a common third working channel therein for the second guide member 102 and the electrode guide needle 103. That is, the second guide member 102 and the electrode guide needle 103 are removably disposed in a common working channel. As a result, the structure of the support catheter can be simplified. The support catheter can be made of any suitable material, for example, it can be made of a biomedical metal material, including but not limited to one or more of stainless steel, synthetic fiber, carbon fiber, titanium alloy, gold, silver, and the like. In order to facilitate bending of the distal end 101-1 of the support catheter, the distal end 101-1 of the support catheter may have a material hardness lower than that of the proximal end 101-2 of the support catheter. In some embodiments, the distal end 101-1 of the support catheter may be configured with a first imaging mark to facilitate locating the first support point a under imaging.

In some embodiments, the second guide member 102 may be supported or tensioned on the blood vessel wall 7 at the second support point b, so that the blood vessel wall 7 at the second support point b is stretched. As a result, the fixed or anchored second support point b is established in the blood vessel channel 6. On the other hand, the tension of the blood vessel wall 7 helps the electrode guide needle 103 to smoothly puncture the blood vessel wall 7 (or, as described below, to pass through the blood vessel wall 7 with the help of the puncture needle 21). In some embodiments, in order to establish the second support point b, the second guide member 102 can be constructed as a stent assembly, which includes a guide wire (not shown), a guide catheter 102-3, and a support stent 102-4, wherein the guide wire is used to guide the guide catheter 102-3, the guide catheter 102-3 is used to guide the support stent 102-4, and the support stent 102-4 is used to support on the blood vessel wall 7. Specifically, the stent assembly can establish the second support point b through the following steps: first, deliver the distal end of the guide wire of the stent assembly to the second support point b through the working channel of the support catheter; then, deliver the distal end of the guide catheter 102-3 of the stent assembly to the second support point b along the guide wire, withdraw the guide wire, but leave the guide catheter 102-3 in place; then, push the support stent 102-4 of the stent assembly to the second support point b, and withdraw the guide catheter 102-3, so that the support stent 102-4 is opened at the second support point b and supports on the blood vessel wall 7.

The guide wire may have any suitable elongated structure. In some embodiments, the guide wire may have a gradually changing outer diameter, for example, it may gradually become thinner from the proximal end to the distal end. The guide wire may be made of a metal material, including but not limited to stainless steel, aluminum alloy, tungsten, and the like. Furthermore, to facilitate pushing, the proximal end of the guide wire may have a material hardness greater than that of the distal end of the guide wire. Additionally, the distal end of the guide wire may further be shaped.

In some embodiments, in order to deliver the distal end of the guide catheter 102-3 to the second support point b along the guide wire, the guide catheter 102-3 can sleeve the guide wire and can move along the guide wire. In order to sleeve the guide wire, the inner diameter of the guide catheter 102-3 may be configured to be greater than or equal to the outer diameter of the guide wire. Furthermore, to facilitate pushing, the proximal end of the guide catheter 102-3 may have a material hardness greater than that of the distal end of the guide catheter 102-3. The guide catheter 102-3 can be made of any suitable material, for example, it can be made of one or more of the following materials: acrylonitrile butadiene styrene (ABS), polyethylene (PE), polyvinyl chloride (PVC), polypropylene (PP), polymethylpentene (PMP), polymethyl methacrylate (PMMA); polycarbonate (PC), polyphenylene oxide (PPO), modified phenylene oxide (modified PPO), polyphenylene ether (PPE); polyimide (PI), polybenzimidazole (PBI); polyphenylene sulfide (PPS), polyetheretherketone (PEEK); fluorinated ethylene-propylene (FEP), ethylene-chlorotrifluoroethylene (ECTFE), ethylene, ethylene-tetrafluoroethylene (ETFE), polychlorotrifluoroethylene (PCTFE), polytetrafluoroethylene (PTFE), expanded polytetrafluoroethylene (ePTFE), polyvinylidene fluoride (PVDF); elastomeric silicone polymer, polyether front segment amide or thermoplastic copolyether (PEBAX); metal (such as stainless steel and nickel titanium alloy), etc. The distal end of the guide catheter 102-3 may be configured with a second imaging marker to facilitate positioning under imaging.

In some embodiments, in order for the support stent 102-4 to be able to automatically expand after the guide catheter 102-3 is withdrawn, the support stent 102-4 may be constructed as a self-expanding stent. The self-expanding stent may include a proximal first push rod and a distal stent body 102-4-1, wherein the first push rod is used to push the stent body 102-4-1 through the guide catheter 102-3, and the stent body 102-4-1 can radially open and support on the blood vessel wall 7 by relying on a self-expanding force after leaving the guide catheter 102-3. In order for the stent body 102-4-1 to be able to leave the guide catheter 102-3, the guide catheter 102-3 may be withdrawn, or the stent body 102-4-1 may be further pushed distally by the first push rod. In order to generate the self-expanding force, the stent body 102-4-1 of the self-expanding stent may be made of a memory alloy material. In some embodiments, as shown in FIG. 2, the support body 102-4-1 may include a plurality of closed-loop network units and a plurality of open-loop network units. In order for the self-expanding stent to pass smoothly through the guide catheter 102-3, the outer diameter of the self-expanding stent in a compressed state may be configured to be smaller than or substantially equal to the inner diameter of the guide catheter 102-3. A third imaging mark may be configured on the stent body 102-4-1 of the self-expanding stent to facilitate the positioning of the second support point b under imaging.

FIGS. 4A-4C respectively show an electrode guide needle 103 of an interventional device 100 used for implanting an electrode 32 into the brain through a blood vessel according to one or more exemplary embodiments of the present disclosure. It should be noted here that the embodiments of FIGS. 4A to 4C differ only in the matching portion 103-7, and therefore, in order to facilitate readers' understanding and comparison, the same reference numerals are used in FIGS. 4A to 4C.

In some embodiments, as shown in FIG. 2 and FIG. 4A to FIG. 4C, the electrode guide needle 103 can be removably disposed in the working channel of the support catheter, and can include a proximal second push rod 103-6 and a distal matching portion 103-7 for the electrode 32. In order to achieve the matching between the electrode 32 and the electrode guide needle 103, a hole may be configured on the electrode 32; the matching portion 103-7 of the electrode guide needle 103 may be configured as a pointed end, and the pointed end may pass through the hole of the electrode 32. In some embodiments, as shown in FIGS. 4A and 4B, the pointed end may have a stepped structure so that the pointed end can carry the electrode 32 when it moves distally, and the electrode 32 can fall off the pointed end when the pointed end is retracted proximally after being implanted in the brain. Instead of the stepped structure, as shown in FIG. 4C, the pointed end may also have a cone-shaped structure. The electrode guide needle 103 can be made of any suitable metal material, for example, it can be made of a biomedical metal material, including but not limited to stainless steel or tungsten. In some embodiments, as shown in FIG. 2, the electrode guide needle 103 may be equipped with a puncture catheter 103-5 for guiding the puncture needle 103 to a target puncture area. In some embodiments, the puncture needle 103 can first be guided to a position about 0.5 mm, 1 mm, 1.5 mm, or 2 mm near the target puncture area with the help of the puncture catheter 103-5 and then stop moving. Then, the puncture needle 103can be made to puncture the blood vessel wall 7 by quickly pushing the second push rod 103-6 of the puncture needle 103. In some embodiments, as shown in FIG. 2, the puncture catheter 103-5 can sleeve the puncture needle 103 and can puncture the blood vessel wall 7. The puncture catheter 103-5 may be formed of the same material as that constructed as the first guide 101 of the support catheter.

**In** some embodiments, the reference numeral 103-5 in FIG. 2 may be a puncture needle provided for the electrode guide needle 103. The puncture needle has a pointed end capable of puncturing a blood vessel wall and has a cavity inside thereof to accommodate an electrode guide needle 103 carrying an electrode. The puncture needle is used to puncture a blood vessel so that the electrode guide needle 103 carrying an electrode can directly pass through the puncture hole, thereby protecting the electrode guide needle 103 and the electrode it carries. The puncture needle can be made of any suitable material, for example, it can be made of a biomedical metal material, including but not limited to one or more of stainless steel, synthetic fiber, carbon fiber, titanium alloy, gold, silver, tungsten, and the like. The puncture needle and the electrode guide needle 103 together constitute a puncture kit. **In** some embodiments, the puncture needle can also be equipped with a needle sheath. The needle sheath is disposed outside the puncture needle to protect the pointed end of the puncture needle to prevent the pointed end of the puncture needle from puncturing the catheter (e.g., the first guide member 101).

FIGS. 5A-5C respectively show several exemplary occluders 103-8 equipped for a puncture kit in an interventional device 100 for implanting an electrode 32 into the brain through a blood vessel according to one or more exemplary embodiments of the present disclosure. It should be noted here that the embodiments of FIGS. 5A to 5C differ only in the occluding portion 103-8-2, and therefore, in order to facilitate readers' understanding and comparison, the same reference numerals are used in FIGS. 5A to 5C.

In some embodiments, the puncture kit may be equipped with an occluder 103-8 for occluding the punctured blood vessel wall 7. As a result, it can be ensured that blood and embolism do not penetrate into the non-vascular area through the punctured blood vessel wall 7. In some embodiments, the occluder 103-8 can be constructed as a self-expanding occluder, which includes a proximal third push rod 103-8-1 and a distal occluding structure 103-8-2, wherein the illustrated third push rod 103-8-1 is used to push the occluding structure 103-8-2 through the puncture catheter 103-5, and the occluding structure 103-8-2 can radially open and adhere to the punctured blood vessel wall 7 by relying on a self-expanding force after leaving the puncture catheter 103-5. In order to allow the occluding structure 103-8-2 to leave the puncture catheter 103-5, the puncture catheter 103-5 can be withdrawn, or the occluding structure 103-8-2 can be pushed distally by the third push rod 103-8-1. In some embodiments, the occluding structure 103-8-2 may adhere to the punctured blood vessel wall 7 on the inner side (not shown) or the outer side (see FIG. 2) of the blood vessel wall 7. In order to perform occlusion on the outer side of the blood vessel wall 7, the puncture catheter 103-5 can puncture the blood vessel wall 7 as shown in FIG. 2. In order to form the self-expanding force, the occluding structure 103-8-2 of the self-expanding occluder may be made of a memory alloy material. In some embodiments, as shown in FIGS. 5A to 5C , the occluding structure 103-8-2 of the occluder 103-8 may have a flower-shaped structure, wherein it is a three-petal flower shape in FIG. 5A, a four-petal flower shape in FIG. 5B, and a five-petal flower shape in FIG. 5C. It is understandable that these different flower shapes are merely exemplary and not restrictive, and the occluding structure 103-8-2 of the occluder 103-8 may also have other suitable shapes, such as umbrella, disc, ring, cylinder, or funnel-shaped structures. Furthermore, in some embodiments, an electric release portion may be constructed at the distal end of the third push rod 103-8-1 to cause the occluding structure 103-8-2 to fall off from the third push rod 103-8-1 when it is powered on. Thus, the third push rod 103-8 can be withdrawn outside the blood vessel.

According to the interventional device 100 of various embodiments of the present disclosure, the electrode 32 is implanted into the brain by establishing an electrode implantation path in a blood vessel, thereby not only alleviating the trauma suffered by a patient, but also reducing the workload of doctors. Furthermore, since blood vessels are distributed in almost all areas of the brain, the electrode 32 can be implanted in almost any area of the brain through corresponding blood vessels. In addition, according to the interventional device 100 disclosed in the present disclosure, the insertion direction or the target puncture area of the puncture kit can be set or adjusted in a targeted manner by determining or adjusting the positions of the first support point a and the second support point b, thereby providing a relatively stable puncture track for the puncture kit of the interventional device 100 and thus improving the accuracy of the implantation of the electrode 32. In addition, the interventional device 100 according to the present disclosure may be equipped with an occluder 103-8 for occluding the punctured blood vessel wall 7, thereby ensuring that the blood and embolism will not penetrate into the non-vascular area through the punctured blood vessel wall 7.

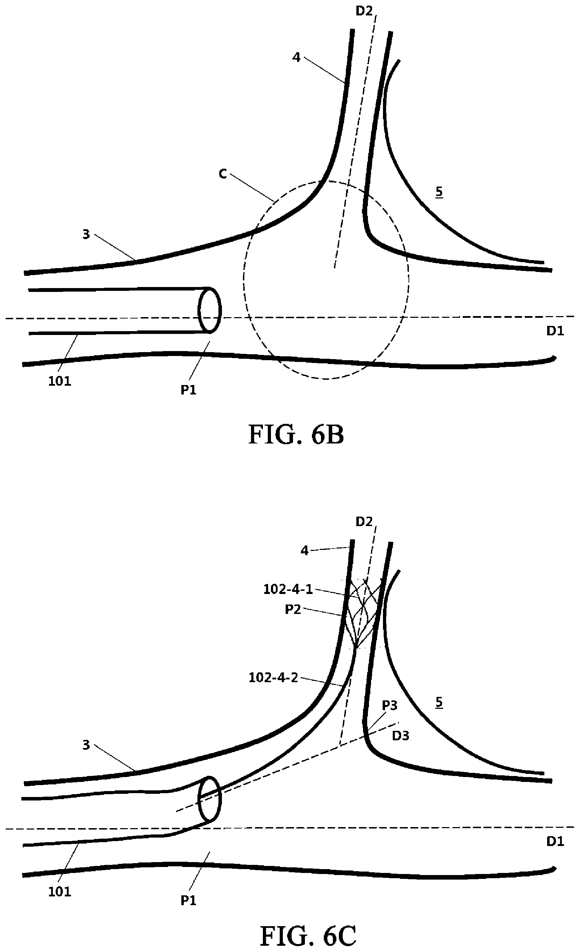

FIGS. 6A-6G are schematic diagrams that schematically show the use of an interventional device for implanting an electrode into the brain through a blood vessel according to other exemplary embodiments of the present disclosure. As shown in FIG. 6A, the direction in which the main blood vessel 3 extends near the connection C between the main blood vessel 3 and the branch blood vessel 4 is a first direction D1, and the direction in which the branch blood vessel 4 extends near the connection C is a second direction D2. As shown in FIG. 6B, the first guide member 101 (also referred to as the "first catheter 101" in this exemplary embodiment) travels in the lumen of the main blood vessel 3, and its distal end can reach and float at a first position P1 in the main blood vessel 3. The first position P1 is located near the connection C and may correspond to the first support point a in the above embodiment. Being "near" the connection C means that it cannot be too far from the connection C, so that the first position P1 is suitable for other components to enter the branch blood vessel 4 from the first position P1 through the connection C. At this time, due to the mechanical strength of the first catheter 101 itself and the blood flow in the lumen of the main blood vessel 3, the opening of the distal end of the first catheter 101 is substantially facing the first direction D1. The outer diameter of the distal end of the first catheter 101 is smaller than the inner diameter of the main blood vessel 3, so that the distal end of the first catheter 101 can float, to a limited extent, radially in the main blood vessel 3 so as to change the direction of the opening of the distal end of the first catheter 101 in subsequent steps.

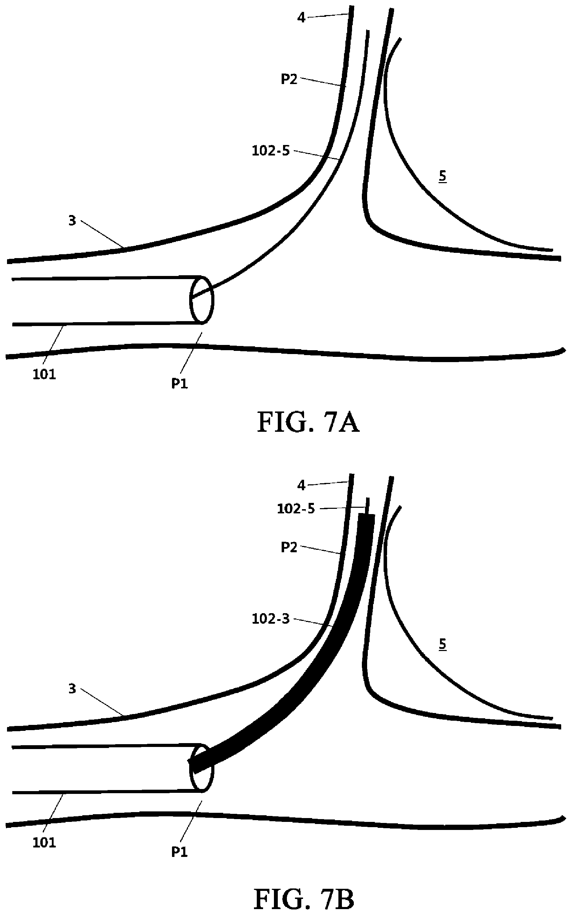

As shown in FIG. 6C, the stent 102-4-1 (i.e., the stent body 102-4-1 in the above embodiment) at the distal end of the stent assembly can reach the distal end of the first catheter 101 along the lumen of the first catheter 101, pass through the connection C and enter the branch blood vessel 4, and be fixed at a second position P2 in the branch blood vessel 4. The first position P2 may also be located near the connection C and may correspond to the second support point b in the above embodiment. The operation process of fixing the bracket 102-4-1 to the second position P2 can refer to the operation process described below in conjunction with FIGS. 7A to 7D. The stent assembly further includes a stent push rod 102-4-2 connected to the stent 102-4-1. After the stent 102-4-1 is fixed at the second position P2, the stent push rod 102-4-2 connected to the rear of the stent 102-4-1 is able to extend between the first position P1 and the second position P2 along the branch direction of the blood vessel, so that the opening at the distal end of the first catheter 101 is deflected toward the second direction D2 under the action of the stent push rod 102-4-2. Thus, the opening of the distal end of the first conduit 101 faces a third direction D3, and the third direction D3 is in an angle area between the first direction D1 and the second direction D2 (which may be similar to the angle area h in the above embodiment).

The third catheter 103-5 is inserted from the first catheter 101, so that the distal end of the third catheter 103-5 reaches and extends out of the distal end of the first catheter 101 along the lumen of the first catheter 101 to approach the puncture position P3 on the blood vessel wall. Then, before the third catheter 103-5 punctures the blood vessel wall, the electrode guide needle 103 can be inserted into the third catheter 103-5, and the distal end of the electrode guide needle 103 can be made to approach the distal end of the third catheter 103-5 along the lumen of the third catheter 103-5. After the distal end of the electrode guide needle 103 approaches the distal end of the third catheter 103-5 (which may be within the lumen of the third catheter 103-5 without extending out of the lumen, or may be slightly extending out of the lumen of the third catheter 103-5), the distal end of the electrode guide needle 103 does not continue to move forward to avoid puncturing the blood vessel wall at this point; moreover, as shown in FIG. 6D, the distal end of the third catheter 103-5 is made to puncture the blood vessel wall at the puncture position P3 and reaches outside the blood vessel without entering the brain, and the electrode guide needle 103 is made to continue to move along the lumen of the third catheter 103-5 and passes through the distal end of the third catheter 103-5 to enter the brain 5, thereby implanting the carried electrode 32 into the brain 5.

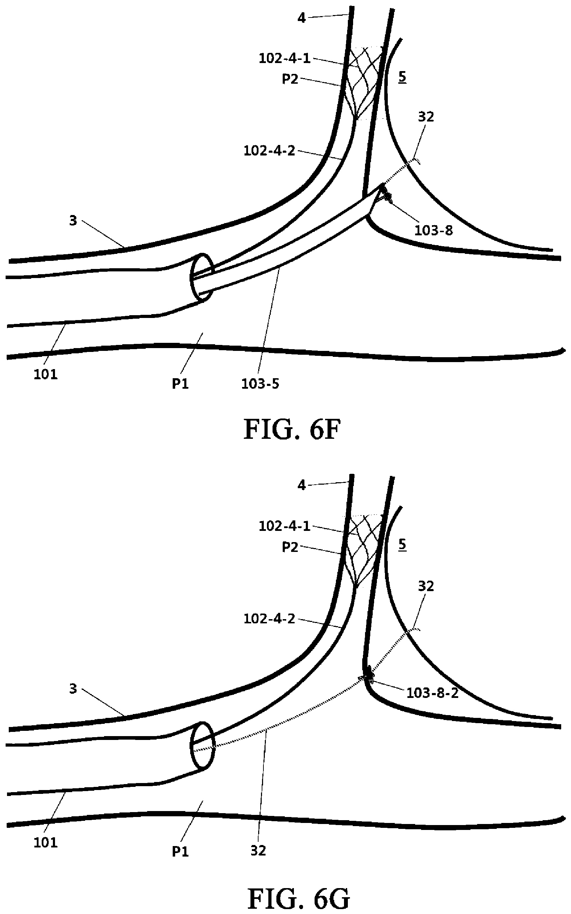

After the electrode 32 is implanted in the brain 5, as shown in FIG. 6F, the electrode guide needle 103 is quickly withdrawn along the lumen of the third catheter 103-5, and the occluder assembly 103-8 is sent into the third catheter 103-5, so that the occluding structure 103-8-2 included in the occluder assembly 103-8 for occluding the puncture hole on the blood vessel wall reaches the distal end of the third catheter 103-5 along the lumen of the third catheter 103-5, and opens after extending out of the distal end of the third catheter 103-5. As shown in FIG. 6G, the third catheter 103-5 is withdrawn, and the occluder push rod connected to the occluding structure 103-8-2 included in the occluder assembly 103-8 is withdrawn, so that the occluding structure 103-8-2 is attached to the blood vessel wall to occlude the puncture hole.

FIGS. 7A-7F are schematic diagrams that schematically show the use of an interventional device for implanting an electrode into the brain through a blood vessel according to other exemplary embodiments of the present disclosure. In these exemplary embodiments, the interventional device may perform the operations described above in conjunction with FIG. 6A and FIG. 6B. After the distal end of the first catheter 101 reaches the first position P1, a guide wire 102-5 can be extended into the first catheter 101, and the guide wire 102-5 can be made to reach the distal end of the first catheter 101 along the lumen of the first catheter 101, pass through the connection C and enter the branch blood vessel 4, and reach the vicinity of the second position P2. Although in the example shown in FIG. 7A, the distal end of the guide wire 102-5 reaches a position in the branch blood vessel 4 that exceeds the second position P2 (i.e., farther than the second position P2), those skilled in the art should understand that in other examples, the distal end of the guide wire 102-5 may reach exactly the second position P2 or reach a position in the branch blood vessel 4 that is slightly closer than the second position P2. The guide wire 102-5 is relatively thin, and its distal end has good shaping ability, so that it can easily enter the branch blood vessel 4 from the main blood vessel 3 through the connection C.

Afterwards, as shown in FIG. 7B, the second catheter 102-3 sleeves the guide wire 102-5 and extends into the first catheter 101, so that the distal end of the second catheter 102-3 can reach at least the second position P2 along the extension trajectory of the guide wire 102-5, that is, reach the second position P2 or a position in the branch blood vessel 4 that is farther than the second position P2. Those skilled in the art should understand that in other embodiments, under the guide by the guide wire 102-5, the distal end of the second catheter 102-3 may move slightly farther than the distal end of the guide wire 102-5.

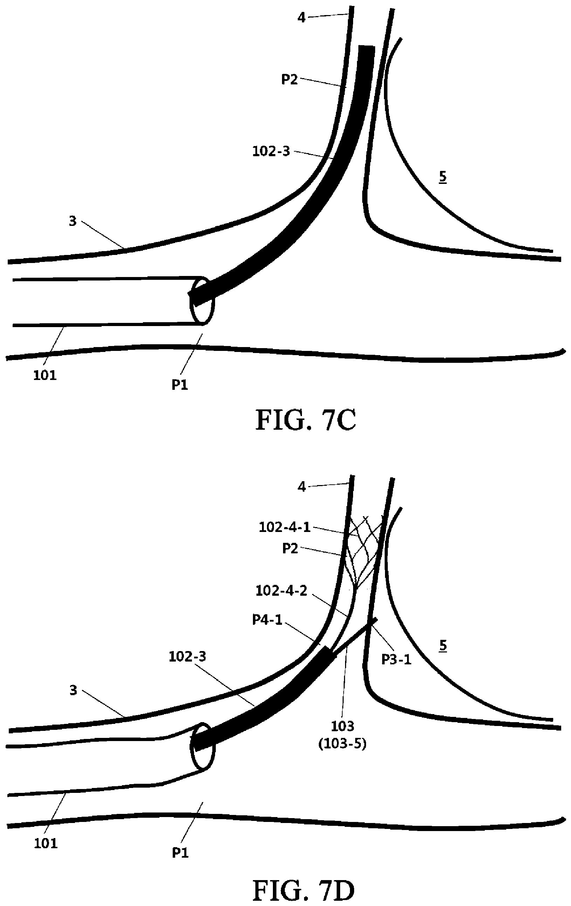

As shown in FIG. 7C, after the distal end of the second catheter 102-3 reaches at least the second position P2, the guide wire 102-5 is withdrawn along the lumen of the second catheter 102-3 so that the stent assembly can enter the second catheter 102-3. The stent assembly is introduced into the second catheter 102-3, and the stent 102-4-1 is moved along the lumen of the second catheter 102-3 to reach the second position P2. After the stent 102-4-1 reaches the second position P2, the second catheter 102-3 is withdrawn along the trajectory of the stent push rod 102-4-2 so that the stent 102-4-1 is exposed outside the second catheter 102-3, thereby fixing the stent 102-4-1 at the second position P2 after self-expansion; and the second catheter 102-3 is withdrawn so that the distal end of the second catheter 102-3 reaches and is located at the first specific position P4-1, as shown in FIG. 7D. The first specific position P4-1 corresponds to the first puncture position P3-1. The electrode guide needle 103 is extended into the second catheter 102-3, and the distal end of the electrode guide needle 103 is moved along the lumen of the second catheter 102-3 to reach the distal end of the second catheter 102-3, and continues to move forward after passing the distal end of the second catheter 102-3, so as to puncture the blood vessel wall at the first puncture position P3-1 corresponding to the first specific position P4-1, thereby entering the brain 5 to implant the carried electrode into the brain 5. Those skilled in the art should understand that the third catheter 103-5 can also be inserted into the second catheter 102-3 and puncture the blood vessel wall at the first puncture position P3-1, and then the electrode guide needle 103 can be inserted into the third catheter 103-5 so that the electrode guide needle 103 finally reaches the brain 5.

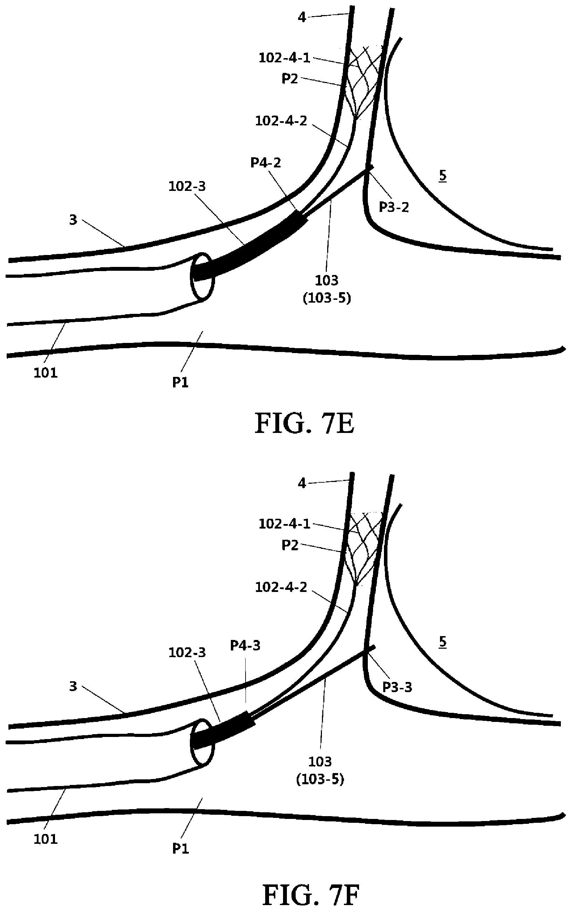

As shown in FIG. 7E, the second catheter 102-3 is retracted again so that the distal end of the second catheter 102-3 reaches and is located at the second specific position P4-2, which corresponds to the second puncture position P3-2. The electrode guide needle 103 (or the third catheter 103-5) is extended into the second catheter 102-3, and the distal end of the electrode guide needle 103 is moved along the lumen of the second catheter 102-3 to puncture the blood vessel wall at the second puncture position P3-2. As shown in FIG. 7F, the second catheter 102-3 is retracted again so that the distal end of the second catheter 102-3 reaches and is located at the third specific position P4-3, and the third specific position P4-3 corresponds to the second puncture position P3-3. The electrode guide needle 103 (or the third catheter 103-5) is extended into the second catheter 102-3, and the distal end of the electrode guide needle 103 is moved along the lumen of the second catheter 102-3 to puncture the blood vessel wall at the third puncture position P3-3.

In the presence of the third catheter 103-5, the occluder assembly is used in the same manner as in the above-described embodiment. In the absence of the third catheter 103-5, such as the case shown in FIGS. 7D to 7F, the occluder assembly can be extended into the second catheter 102-3 after the electrode guide needle 103 completes the puncture and before its distal end is withdrawn into the blood vessel, and the occluding structure can be made to reach the distal end of the second catheter 102-3 along the lumen of the second catheter 102-3 and continue to move forward after passing the distal end of the second catheter 102-3 to reach the vicinity of the puncture position (the corresponding one among P3-1 to P3-3). After the distal end of the electrode guide needle 103 is withdrawn into the blood vessel, the occluding structure is quickly passed through the blood vessel wall from the puncture hole at the puncture position, and after reaching outside the blood vessel, it opens and adheres to the blood vessel wall to occlude the puncture hole. Compared with the occlusion method shown in FIG. 6F, such use of the occluder further shortens the exposure time of the puncture hole (the time from when the electrode guide needle 103 is withdrawn from the puncture hole to when the occluding structure occludes the puncture hole).

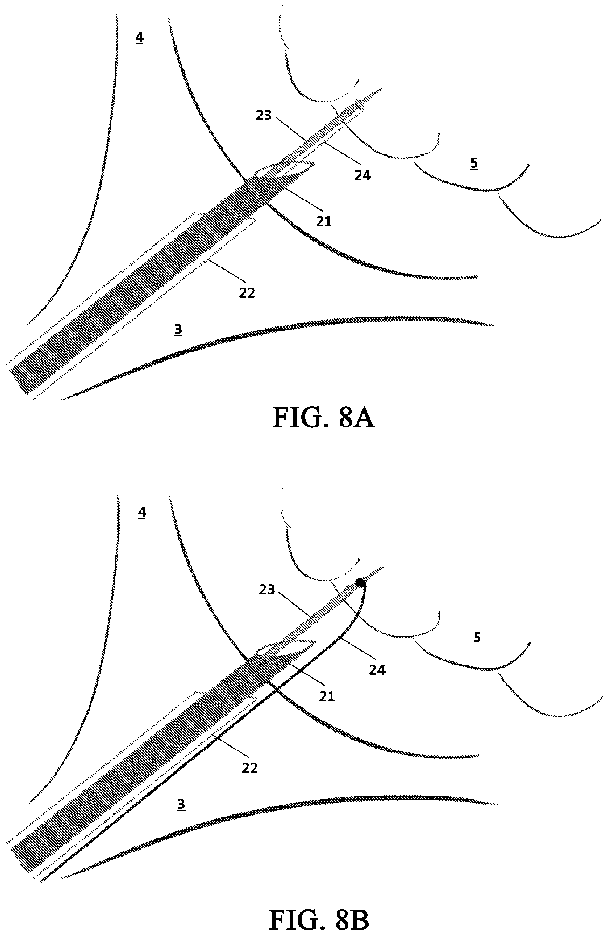

In some embodiments, the electrode guide needle 103 in the embodiment depicted in FIGS. 7A to 7F may be implemented as a puncture kit as shown in FIGS. 8A and 8B. The puncture kit includes a puncture needle 21, a needle sheath 22, and an electrode guide needle 23 for carrying an electrode 24. The electrode guide needle 103 in the embodiment depicted in FIGS. 6A to 6G can be implemented as the electrode guide needle 23 in the puncture kit as shown in FIGS. 8A and 8B, and the third catheter 103-5 can be implemented as the puncture needle 21 in the puncture kit as shown in FIGS. 8A and 8B. The puncture needle 21 has a pointed end at the distal end for puncturing the blood vessel wall and is configured as a cavity inside. The puncture needle 21 can be made of any suitable metal material, for example, it can be made of a biomedical metal material, including but not limited to one or more of stainless steel, synthetic fiber, carbon fiber, titanium alloy, gold, silver, and the like. The needle sheath 22 sleeves the puncture needle 21 to protect the pointed end of the puncture needle 21 to prevent the pointed end of the puncture needle 21 from puncturing a catheter outside the puncture needle 21. The needle sheath 22 may be made of a material softer than metals, such as the same material as that of the catheter. Furthermore, the needle sheath 22 may also be made of a metal, but may be constructed without a sharp tip to avoid puncturing the catheter. The electrode guide needle 23 is located in the cavity of the puncture needle, and its distal end has a matching portion constructed to carry an electrode. The electrode 24 carried by the electrode guide needle 23 may be carried inside the puncture needle 21 (as shown in FIG. 8A), and may also be carried outside the puncture needle 21 and the needle sheath 22 (as shown in FIG. 8B).

The operation of the puncture kit is described below with reference to the embodiment depicted in FIG. 7A to FIG. 7F. The puncture kit is extended into the second catheter 102-3, and the distal ends of the puncture needle 21, the needle sheath 22, and the electrode guide needle 23 in the puncture kit are moved together along the lumen of the second catheter 102-3 to reach the distal end of the second catheter 102-3. After passing through (or referred to as extending out of) the distal end of the second catheter 102-3, the distal end of the needle sheath 22 stops moving, while the distal ends of the puncture needle 21 and the electrode guide needle 23 continue to move together to extend out of the needle sheath 22 and approach the puncture position. After reaching the puncture position, the distal end of the puncture needle 21 punctures the blood vessel wall at the puncture position and reaches outside the blood vessel. The distal end of the puncture needle 21 stops moving after reaching outside the blood vessel, while the distal end of the electrode guide needle 23 continues moving in the cavity of the puncture needle 21 and passes through the distal end of the puncture needle 21 to enter the brain, thereby implanting the carried electrode into the brain.

In the above description of the various embodiments of the present disclosure, a scene near the connection between the main blood vessel 3 and the branch blood vessel 4 extending from the main blood vessel 3 as a branch is used to describe the operation of the interventional device 100. In fact, the interventional device 100 according to any of the above embodiments of the present disclosure can also operate in the scenario that is not a connection between the main blood vessel 3 and the branch blood vessel 4. For example, in other embodiments, the operation scenario of the interventional device 100 may be near the connection between a first blood vessel segment and a second blood vessel segment, where the first blood vessel segment extends along a first direction and the second blood vessel segment extends along a second direction different from the first direction. For example, the first blood vessel segment and the second blood vessel segment may be connected to form a portion of a blood vessel with an arc curve and no branches.

On the other hand, the present disclosure further provides a medical apparatus, which may include the interventional device 100 according to any of the above embodiments of the present disclosure, and will not be described in detail herein.

The words "left," "right," "front," "back," "top," "bottom," "upper," "lower," "high," "low," and the like in the specification and claims, if present, are used for descriptive purposes and not necessarily for describing unchanging relative positions. It should be understood that the terms so used are interchangeable under appropriate circumstances such that the embodiments of the disclosure described herein are, for example, capable of operation in other orientations than those illustrated or otherwise described herein. For example, when the device in the figures is turned over, features previously described as "above" other features may now be described as "below" the other features. The device may be otherwise oriented (rotated by 90 degrees or at other orientations), and the relative spatial relationships should be interpreted accordingly.

In the specification and claims, when an element is referred to as being "on," "attached" to, "connected" to, "coupled to," "coupled to," or "contacting" another element, the element may be directly on, directly attached to, directly connected to, directly coupled to, directly coupled to, or directly contacting another element, or one or more intermediated elements may be present. In contrast, when an element is referred to as being "directly on," "directly attached to," "directly connected to," "directly coupled to," "directly coupled to," or "directly contacting" another element, there will be no intermediated elements present. In the specification and claims, a feature being arranged "adjacent" to another feature may mean that one feature has a portion overlapping with an adjacent feature or a portion located above or below an adjacent feature.

As used herein, the word "exemplary" means "serving as an example, instance, or illustration" rather than as a "model" to be exactly copied. Any implementation described herein as exemplary is not necessarily to be construed as preferred or advantageous over other implementations. Furthermore, this disclosure is not intended to be bound by any expressed or implied theory presented in the technical field, background, summary, or detailed description of the invention.

As used herein, the term "substantially" is meant to include defects caused by design or manufacturing, device or component tolerances, environmental impacts, and/or any minor variations caused by other factors. The term "substantially" also allows for deviations from the perfect or ideal case due to parasitic effects, noise, and other practical considerations that may be present in actual implementations.

In addition, "first," "second," and other similar terms may also be used herein for reference purposes only, and thus are not intended to be limiting. For example, the terms "first," "second," and other such numerical terms when referring to structures or elements do not imply a sequence or order unless clearly indicated by the context.

It should also be understood that when the term "include/comprise" is used herein, it indicates the presence of the specified features, whole, steps, operations, units, and/or components, but does not exclude the presence or addition of one or more other features, whole, steps, operations, units, and/or components and/or combinations thereof.

In the present disclosure, the term "provide" is used in a broad sense to cover all ways of obtaining an object, and thus "providing an object" includes but is not limited to "purchasing," "preparing/manufacturing," "arranging/setting up," "installing/assembling," and/or "ordering" an object.

As used herein, the term "and/or" includes any and all combinations of one or more of associated listed items. The terms used herein are for the purpose of describing particular embodiments only and are not intended to limit the present disclosure. As used herein, the singular forms "a," "an," and "the" are intended to include the plural forms as well, unless otherwise clearly indicated in the context.

Those skilled in the art should appreciate that the boundaries between the above operations are merely illustrative. A plurality of operations may be combined into a single operation, a single operation may be distributed among additional operations, and operations may be performed at least partially overlapping in time. Moreover, alternative embodiments may include a plurality of instances of a particular operation, and the order of operations may be altered in other various embodiments. However, other modifications, variations, and alternatives are also possible. Aspects and elements of all the above-disclosed embodiments may be combined in any manner and/or in combination with aspects or elements of other embodiments to provide a plurality of additional embodiments. Therefore, the specification and the accompanying drawings are to be seen in an illustrative rather than a restrictive sense.

Although some specific embodiments of the present disclosure have been described in detail through examples, those skilled in the art should understand that the above examples are only for illustration rather than for limiting the scope of the present disclosure. The various embodiments disclosed herein may be combined in any manner without departing from the spirit and scope of the present disclosure. Those skilled in the art should further appreciate that various modifications may be made to the embodiments without departing from the scope and spirit of the present disclosure. The scope of the present disclosure is defined by the appended claims.

## Claims

1. An interventional device for implanting an electrode into the brain through a blood vessel, the blood vessel comprising a blood vessel channel and a blood vessel wall, **characterized in that** the interventional device comprises:
a first guide member, wherein a distal end of the first guide member is capable of moving distally in the blood vessel channel and establishing a first support point in the blood vessel channel;
a second guide member, wherein a distal end of the second guide member is capable of passing through the first guide member, moving distally in the blood vessel channel, and establishing a second support point in the blood vessel channel, so that the second guide member extends in an arc shape between the first support point and the second support point; and
a puncture kit, wherein the puncture kit is used to carry an electrode, and a distal end of the puncture kit can pass through the first guide, move distally in a blood vessel channel, and puncture the blood vessel wall in a target puncture area between the first support point and the second support point, thereby implanting the carried electrode into the brain.

2. The interventional device for implanting an electrode into the brain through a blood vessel according to claim 1, **characterized in that** the blood vessel comprises a main blood vessel and a branch blood vessel, the first support point is located in the main blood vessel, and the second support point is located in the branch blood vessel.

3. The interventional device for implanting an electrode into the brain through a blood vessel according to claim 2, **characterized in that** the target puncture area is located in a transition area between the main blood vessel and the branch blood vessel.

4. The interventional device for implanting an electrode into the brain through a blood vessel according to any one of claims 1 to 3, **characterized in that** the first guide member is constructed as a support catheter, and the outer diameter of the distal side of the support catheter is substantially equal to the inner diameter of the blood vessel at the first support point, so as to establish the first support point in the blood vessel channel.

5. The interventional device for implanting an electrode into the brain through a blood vessel according to claim 4, **characterized in that** the support catheter comprises a first working channel for the second guide member and has a second working channel for the puncture kit.

6. The interventional device for implanting an electrode into the brain through a blood vessel according to claim 4, **characterized in that** a distal end of the support catheter has a material hardness lower than that of the proximal end of the support catheter.

7. The interventional device for implanting an electrode into the brain through a blood vessel according to claim 4, **characterized in that** a distal end of the support catheter is constructed with a first imaging mark.

8. The interventional device for implanting an electrode into the brain through a blood vessel according to any one of claims 1 to 3, **characterized in that** the second guide member is constructed as a stent assembly, and the stent assembly includes a guide wire, a guide catheter, and a support stent, wherein the guide wire is used to guide the guide catheter, the guide catheter is used to guide the support stent, and the support stent is used to support on the blood vessel wall.

9. The interventional device for implanting an electrode into the brain through a blood vessel according to claim 8, **characterized in that** a distal end of the guide wire can be shaped.

10. The interventional device for implanting an electrode into the brain through a blood vessel according to claim 8, **characterized in that** the guide wire gradually becomes thinner from the proximal end to the distal end.

11. The interventional device for implanting an electrode into the brain through a blood vessel according to claim 8, **characterized in that** the guide wire is made of a metal material.

12. The interventional device for implanting an electrode into the brain through a blood vessel according to claim 8, **characterized in that** the guide catheter sleeves the guide wire and can move along the guide wire.

13. The interventional device for implanting an electrode into the brain through a blood vessel according to claim 8, **characterized in that** the inner diameter of the guide catheter is configured to be greater than or equal to the outer diameter of the guide wire.

14. The interventional device for implanting an electrode into the brain through a blood vessel according to claim 8, **characterized in that** the distal end of the guide catheter is constructed with a second imaging mark.

15. The interventional device for implanting an electrode into the brain through a blood vessel according to claim 8, **characterized in that** the proximal end of the guide catheter has a material hardness greater than that of the distal end of the guide catheter.

16. The interventional device for implanting an electrode into the brain through a blood vessel according to claim 8, **characterized in that** the guide catheter is made of one or more of the following materials: acrylonitrile butadiene styrene (ABS), polyethylene (PE), polyvinyl chloride (PVC), polypropylene (PP), polymethylpentene (PMP), polymethyl methacrylate (PMMA); polycarbonate (PC), polyphenylene oxide (PPO), modified phenylene oxide (modified PPO), polyphenylene ether (PPE); polyimide (PI), polybenzimidazole (PBI); polyphenylene sulfide (PPS), polyetheretherketone (PEEK); fluorinated ethylene-propylene (FEP), ethylene-chlorotrifluoroethylene (ECTFE), ethylene, ethylene-tetrafluoroethylene (ETFE), polychlorotrifluoroethylene (PCTFE), polytetrafluoroethylene (PTFE), expanded polytetrafluoroethylene (ePTFE), polyvinylidene fluoride (PVDF); elastomeric silicone polymer, polyether front segment amide or thermoplastic copolyether (PEBAX); and metals.

17. The interventional device for implanting an electrode into the brain through a blood vessel according to claim 8, **characterized in that** the support stent is constructed as a self-expanding stent, and the self-expanding stent includes a proximal first push rod and a distal stent body, the first push rod is used to push the stent body through the guide catheter, and the stent body can radially open and support on the blood vessel wall by relying on a self-expanding force after leaving the guide catheter.

18. The interventional device for implanting an electrode into the brain through a blood vessel according to claim 17, **characterized in that** the stent body of the self-expanding stent can leave the guide catheter by withdrawing the guide catheter or by pushing the stent body distally by the first push rod.

19. The interventional device for implanting an electrode into the brain through a blood vessel according to claim 17, **characterized in that** the stent body of the self-expanding stent is made of a memory alloy material.

20. The interventional device for implanting an electrode into the brain through a blood vessel according to claim 17, **characterized in that** the stent body of the self-expanding stent is constructed with a third imaging mark.

21. The interventional device for implanting an electrode into the brain through a blood vessels according to claim 17, **characterized in that** the stent body of the self-expanding stent comprises a plurality of closed-loop mesh units and a plurality of open-loop mesh units.

22. The interventional device for implanting an electrode into the brain through a blood vessel according to claim 17, **characterized in that** the outer diameter of the self-expanding stent in a compressed state is smaller than or equal to the inner diameter of the guide catheter.

23. The interventional device for implanting an electrode into the brain through a blood vessel according to any one of claims 1 to 3, **characterized in that** the puncture kit comprises: a puncture needle having a pointed end at the distal end for puncturing and configured as a cavity inside, a needle sheath sleeving the puncture needle for protecting the pointed end of the puncture needle, and an electrode guide needle located in the cavity of the puncture needle, wherein the distal end of the electrode guide needle has a matching portion constructed to carry an electrode.

24. The interventional device for implanting an electrode into the brain through a blood vessel according to claim 23, **characterized in that** the matching portion of the electrode guide needle is constructed as a pointed end, and the pointed end can pass through a hole constructed in the electrode for matching with the electrode guide needle.

25. The interventional device for implanting an electrode into the brain through a blood vessel according to claim 24, **characterized in that** the pointed end has a stepped structure or a cone-shaped structure, so that the pointed end can carry the electrode when it moves distally, and the electrode can fall off from the pointed end when the pointed end is withdrawn proximally after the electrode is implanted in the brain.

26. The interventional device for implanting an electrode into the brain through a blood vessel according to claim 23, **characterized in that** the electrode guide needle is made of a metal material.

27. The interventional device for implanting an electrode into the brain through a blood vessel according to any one of claims 1 to 3, **characterized in that** the puncture kit is equipped with a puncture catheter for guiding the puncture kit to a target puncture area.

28. The interventional device for implanting an electrode into the brain through a blood vessel according to claim 27, **characterized in that** the puncture kit is equipped with an occluder for occluding the punctured blood vessel wall.

29. The interventional device for implanting an electrode into the brain through a blood vessel according to claim 28, **characterized in that** the occluder is constructed as a self-expanding occluder, and the self-expanding occluder comprises a proximal third push rod and a distal occluding structure, the third push rod is used to push the occluding structure through the puncture catheter, and the occluding structure can radially open and adhere to the punctured blood vessel wall by relying on a self-expanding force after leaving the puncture catheter.

30. The interventional device for implanting an electrode into the brain through a blood vessel according to claim 29, **characterized in that** the occluding structure of the occluder is a flower-shaped, umbrella-shaped, disc-shaped, ring-shaped, cylindrical, or funnel-shaped structure.

31. The interventional device for implanting an electrode into the brain through a blood vessel according to claim 29, **characterized in that** an electric release portion is constructed at the distal end of the third push rod to cause the occluding structure to fall off from the third push rod when it is powered on.

32. The interventional device for implanting an electrode into the brain through a blood vessel according to any one of claims 1 to 3, **characterized in that** the electrode is a flexible electrode.

33. The interventional device for implanting an electrode into the brain through a blood vessel according to any one of claims 1 to 3, **characterized in that** the second guide member is supported on the blood vessel wall at the second support point so that the blood vessel wall at the second support point is stretched.

34. The interventional device for implanting an electrode into the brain through a blood vessel according to any one of claims 1 to 3, **characterized in that** the target puncture area is in an angle area formed by the central axis of the first guide member and the second guide member.

35. An interventional device for implanting an electrode into the brain through a blood vessel, the blood vessel comprising a first blood vessel segment and a second blood vessel segment extending in different directions, **characterized in that** the interventional device comprises:
a first guide member, the distal end of which is capable of moving distally in the blood vessel channel of the first blood vessel segment and reaching a first position, the first position being in the first blood vessel segment and being located near a connection between the first blood vessel segment and the second blood vessel segment;
a second guide member, the distal end of which can move distally in the first guide member and continue to move distally after reaching the distal end of the first guide member, so as to pass through the connection between the first and second blood vessel segments and enter the second blood vessel segment, thereby reaching and being fixed at a second position; and
a puncture kit used to carry an electrode, the distal end of which can move distally in a first guide member and continue to move distally after reaching the distal end of the first guide member to puncture a blood vessel wall between the first position and the second position, thereby implanting the carried electrode into the brain.

36. An interventional device for implanting an electrode into the brain through a blood vessel, wherein the blood vessel comprises a first blood vessel segment and a second blood vessel segment connected and extending in different directions, the connection between the first blood vessel segment and the second blood vessel segment is near the brain, and the interventional device comprises:
a first catheter, the first catheter being configured in such a way that its distal end can reach and float at a first position located in the first blood vessel segment, and the opening of the distal end of the first catheter is substantially oriented toward a first direction, the first position being located near the connection, and the first direction being a direction in which the first blood vessel segment extends near the connection;
a stent assembly, the stent assembly comprising a stent located at the distal end and a stent push rod connected to the stent, the stent assembly being configured in such a way that the stent can reach the distal end of the first catheter along the lumen of the first catheter, pass through the connection and enter the second blood vessel segment, and be fixed at a second position located in the second blood vessel segment so that the stent push rod can extend along the branch direction of the blood vessel between the first position and the second position, and the opening of the distal end of the first catheter can be deflected to a second direction under the action of the stent push rod, the second direction being the direction of extension of the second blood vessel segment near the connection;
a third catheter, the third catheter being configured in such a way that its distal end can reach and extend from the distal end of the first catheter along the lumen of the first catheter to approach the puncture position on the blood vessel wall; and
a puncture kit, the puncture kit being used to carry the electrode, and the puncture kit being configured in such a way that its distal end can approach the distal end of the third catheter along the lumen of the third catheter, wherein,
the third catheter is also configured in such a way that after the distal end of the puncture kit approaches the distal end of the third catheter, the distal end of the third catheter can puncture the blood vessel wall at the puncture position and reach outside the blood vessel without entering the brain,
and the puncture kit is also configured in such a way that after the distal end of the third catheter reaches outside the blood vessel, it continues to move along the lumen of the third catheter and passes through the distal end of the third catheter to enter the brain, thereby implanting the carried electrode into the brain.

37. The interventional device according to claim 36, further comprising:
a guide wire, the guide wire being configured in such a way that a distal end thereof is able to reach the distal end of the first catheter along the lumen of the first catheter, pass through the connection, enter the second blood vessel segment, and reach the vicinity of the second position before the stent is fixed at the second position; and
a second catheter, the second catheter being configured in such a way that it is able to sleeve the guide wire and the distal end of the second catheter can reach at least the second position along the extension trajectory of the guide wire after the distal end of the guide wire reaches the vicinity of the second position and before the stent is fixed at the second position, wherein,
the guide wire is further configured to be able to be withdrawn along the lumen of the second catheter after the distal end of the second catheter reaches at least the second position so that the stent assembly can enter the second catheter,
the stent assembly is further configured to be able to enter the second catheter and enable the stent to reach the second position along the lumen of the second catheter,
and the second catheter is further configured to be able to be withdrawn after the stent reaches the second position so as to allow the stent to self-expand and thus be fixed at the second position.

38. The interventional device according to claim 36 or 37, further comprising:
an occluder assembly comprising an occluding structure located at the distal end for occluding a puncture hole on the blood vessel wall and an occluder push rod connected to the occluding structure, wherein,
the puncture kit is further configured to be able to be withdrawn along the lumen of the third catheter after the carried electrode is implanted into the brain so that the occluder assembly can enter the third catheter,
and the occluder assembly is further configured in such a way that the occluding structure can reach the distal end of the third catheter along the lumen of the third catheter, and after extending from the distal end of the third catheter, open and adhere to the blood vessel wall to occlude the puncture hole.

39. The interventional device according to claim 36 or 37, wherein the outer diameter of the distal end of the first catheter is smaller than the inner diameter of the first blood vessel segment.

40. The interventional device according to claim 36 or 37, wherein the first blood vessel segment is a main blood vessel, and the second blood vessel segment is a branch blood vessel extending from the main blood vessel as a branch.

41. An interventional device for implanting an electrode into the brain through a blood vessel, wherein the blood vessel comprises a first blood vessel segment and a second blood vessel segment connected and extending in different directions, the connection between the first blood vessel segment and the second blood vessel segment is near the brain, and the interventional device comprises:
a first catheter, the first catheter being configured in such a way that its distal end can reach and be located at a first position in the first blood vessel segment, the first position being located near the connection;
a stent assembly, the stent assembly comprising a stent located at the distal end and a stent push rod connected to the stent, the stent assembly being configured in such a way that the stent can reach the distal end of the first catheter along the lumen of the first catheter, pass through the connection to enter the second blood vessel segment, and be fixed at a second position in the second blood vessel segment, so that the stent push rod extends between the first position and the second position;
a second catheter, the second catheter being configured in such a way that the second catheter is able to sleeve the stent push rod, and the distal end of the second catheter can reach and be located at a specific position between the first position and the second position; and
a puncture kit, the puncture kit being used to carry the electrode, the puncture kit being configured in such a way that its distal end can reach the distal end of the second catheter along the lumen of the second catheter and continue to move forward after passing the distal end of the second catheter, so as to puncture the blood vessel wall at a puncture position corresponding to the specific position and enter the brain, thereby implanting the carried electrode into the brain.

42. The interventional device according to claim 41, further comprising:
a guide wire, the guide wire being configured in such a way that a distal end thereof is able to reach the distal end of the first catheter along the lumen of the first catheter, pass through the connection, enter the second blood vessel segment, and reach the vicinity of the second position before the stent is fixed at the second position, wherein,
the second catheter is further configured in such a way that it is able to sleeve the guide wire and the distal end of the second catheter can reach at least the second position along the extension trajectory of the guide wire before the stent is fixed at the second position,
the guide wire is further configured to be able to be withdrawn along the lumen of the second catheter after the distal end of the second catheter reaches at least the second position so that the stent assembly can enter the second catheter,
the stent assembly is further configured to be able to enter the second catheter and enable the stent to reach the second position along the lumen of the second catheter,
and the second catheter is further configured to be able to be withdrawn after the stent reaches the second position so as to allow the stent to self-expand and thus be fixed at the second position and enable the distal end of the second catheter to reach and be located at the specific position.

43. An interventional device according to claim 41 or 42, wherein the specific positions are one or more, so that the distal end of the puncture kit can puncture the blood vessel wall and enter the brain at one or more puncture positions corresponding to one or more of the specific positions, thereby implanting one or more carried electrodes into the brain.

44. The interventional device according to claim 41 or 42, further comprising:
an occluder assembly comprising an occluding structure located at the distal end for occluding a puncture hole on the blood vessel wall and an occluder push rod connected to the occluding structure, wherein the occluder assembly is configured in such a way that:
the occluding structure can reach the distal end of the second catheter along the lumen of the second catheter, and continue to move after passing the distal end of the second catheter to reach the vicinity of the puncture position, and
after at least part of the puncture kit is withdrawn into the blood vessel, the occluding structure can puncture through the blood vessel wall from the puncture hole at the puncture position, and after reaching outside the blood vessel, open and adhere to the blood vessel wall to occlude the puncture hole.

45. The interventional device according to claim 41 or 42, wherein the puncture kit comprises: a puncture needle having a pointed end at the distal end for puncturing a blood vessel and configured as a cavity inside, a needle sheath sleeving the puncture needle to protect the pointed end of the puncture needle to prevent the pointed end of the puncture needle from puncturing the second catheter, and an electrode guide needle located in the cavity of the puncture needle, the distal end of the electrode guide needle having a matching portion constructed to carry an electrode.

46. The interventional device according to claim 45, wherein the puncture kit is configured in such a way that:
the distal end of the puncture needle, the distal end of the needle sheath, and the distal end of the electrode guide needle can reach the distal end of the second catheter along the lumen of the second catheter;
after passing the distal end of the second catheter, the distal end of the needle sheath can stop moving, while the distal ends of the puncture needle and the electrode guide needle continue to move to extend out of the needle sheath and approach the puncture position;
the distal end of the puncture needle is capable of puncturing the blood vessel wall at the puncture position and reaching outside the blood vessel without entering the brain;
the distal end of the puncture needle is capable of stopping moving after reaching outside the blood vessel, while the distal end of the electrode guide needle is capable of continuing to move in the cavity of the puncture needle and passing through the distal end of the puncture needle to enter the brain, thereby implanting the carried electrode into the brain.

47. The interventional device according to claim 46, further comprising:
an occluder assembly comprising an occluding structure located at the distal end for occluding a puncture hole on the blood vessel wall and an occluder push rod connected to the occluding structure, wherein,
the electrode guide needle is further configured to be able to be withdrawn along the cavity of the puncture needle after the carried electrode is implanted into the brain so that the occluder assembly can enter the cavity of the puncture needle,
the occluder assembly is further configured in such a way that the occluding structure can reach the distal end of the puncture needle along the cavity of the puncture needle, and after extending from the distal end of the puncture needle, open and adhere to the blood vessel wall to occlude the puncture hole.

48. The interventional device according to claim 41 or 42, wherein the first blood vessel segment is a main blood vessel, and the second blood vessel segment is a branch blood vessel extending from the main blood vessel as a branch.

49. An interventional device for implanting an electrode into the brain through a blood vessel, wherein the blood vessel comprises a first blood vessel segment and a second blood vessel segment connected and extending in different directions, the connection between the first blood vessel segment and the second blood vessel segment is near the brain, and the interventional device comprises:
a first catheter, the first catheter being configured in such a way that its distal end can reach and float at a first position located in the first blood vessel segment, and the opening of the distal end of the first catheter is substantially oriented toward a first direction, the first position being located near the connection, and the first direction being a direction in which the first blood vessel segment extends near the connection;
a stent assembly, the stent assembly comprising a stent located at the distal end and a stent push rod connected to the stent, the stent assembly being configured in such a way that the stent can reach the distal end of the first catheter along the lumen of the first catheter, pass through the connection and enter the second blood vessel segment, and be fixed at a second position located in the second blood vessel segment so that the stent push rod can extend along the branch direction of the blood vessel between the first position and the second position, and the opening of the distal end of the first catheter can be deflected to a second direction under the action of the stent push rod, the second direction being the direction of extension of the second blood vessel segment near the connection; and
an electrode guide needle, the distal end of the electrode guide needle being constructed to carry the electrode, the electrode guide needle being configured in such a way that its distal end can reach and extend from the distal end of the first catheter along the lumen of the first catheter, pass through the blood vessel wall at the puncture position on the blood vessel wall, and enter the brain, thereby implanting the carried electrode into the brain.

50. The interventional device according to claim 49, further comprising:
a puncture needle, the puncture needle being constructed to have a pointed end for puncturing a blood vessel wall and an internal cavity, the puncture needle being configured to be capable of sleeving outside the electrode guide needle and having a distal end that can reach and extend out of the distal end of the first catheter along the lumen of the first catheter together with the distal end of the electrode guide needle, and the distal end of the puncture needle being capable of puncturing the blood vessel wall at the puncture position and reaching outside the blood vessel without entering the brain, and
the electrode guide needle being further configured in such a way that after the distal end of the puncture needle reaches outside the blood vessel, the distal end of the electrode guide needle continues to move along the inner cavity of the puncture needle and passes through the distal end of the puncture needle to enter the brain, thereby implanting the carried electrode into the brain.

51. The interventional device according to claim 50, wherein the puncture needle is provided with a needle sheath, and the needle sheath is constructed to sleeve the puncture needle to protect the pointed end of the puncture needle to prevent the pointed end of the puncture needle from puncturing the first catheter.

52. A medical apparatus, **characterized in that** the medical apparatus comprises the interventional device for implanting an electrode into the brain through a blood vessel according to any one of claims 1 to 51.
